# FASCICULE DE BREVET EUROPEEN

(11) **EP 1 190 036 B1**
(45) Date de publication et mention de la délivrance du brevet: **10.11.2004**
(21) Numéro de dépôt: 00927340.0
(22) Date de dépôt: 15.05.2000
(51) Int. Cl.: C12N 1/12, A01G 33/00, A61K 35/80, A61K 7/48, A23L 3/3463, A01N 63/00, C12N 9/02

(54) **PROCEDE D'OBTENTION, A PARTIR DU MILIEU DE CULTURE DE MICRO-ALGUES, D'UN EXTRAIT THERMOSTABLE POSSEDANT UNE ACTIVITE ANTI-OXYDANTE ET PRO-CICATRISANTE**
VERFAHREN ZUR GEWINNUNG EINES THERMOSTABILEN MIKROALGENEXTRAKTES MIT OXIDATIONSHEMMENDER- UND WUNDHEILENDER AKTIVITÄT
METHOD FOR OBTAINING FROM A CULTURE MEDIUM OF MICROALGAE, A HEAT-STABLE EXTRACT WITH ANTIOXIDANT AND WOUND HEALING ACTIVITY

(30) Priorité: 08.06.1999 FR 9907372
(43) Date de publication de la demande: 27.03.2002
(73) Titulaire: Aquamer (SAEM), 34140 Meze (FR)
(72) Inventeur: POINT, Jacques, F-01100 Oyonnax (FR); BACCOU, Jean-Claude, F-34080 Montpellier (FR); BAROUX, Bruno, F-34580 Saussan (FR)
(74) Mandataire: Roman, Michel
(86) Numéro de dépôt international: PCT/FR2000/001302
(87) Numéro de publication internationale: WO 2000/075282

(56) Documents cités:
- EP-A- 0 437 393
- EP-A- 0 628 629
- FR-A- 2 705 685

## Description

La présente invention a pour objet un procédé d'obtention, à partir du milieu de culture de micro-algues, d'un extrait thermostable possédant une activité anti-oxydante et pro-cicatrisante liée à une teneur élevée en superoxydismutases-like et en polysaccharides sulfatés, et susceptible de trouver des applications dans l'industrie chimique, l'industrie cosmétique, l'industrie pharmaceutique et l'industrie agronomique, ainsi que dans les domaines de la nutraceutique et de la diététique.

De nombreux travaux ont déjà été effectués, dans le domaine cosmétique, pour mettre au point des substances antiradicalaires propres notamment à ralentir le vieillissement cutané. Parallèlement, des recherches ont été menées dans le secteur médical pour obtenir des produits à activité anti-inflammatoire, aptes à être utilisés notamment en rhumatologie et dans le traitement de pathologies liées à des stress oxydatifs et affectant les systèmes digestif et cardio-vasculaire.

Les recherches entreprises ont été délibérément orientées ces dernières années vers le secteur végétal afin d'éviter les substances infectieuses qui risquent de se trouver dans les extraits animaux. Parmi ces recherches, la culture de micro-algues a conduit à l'obtention de produits intéressants à partir de la biomasse algale.

Le brevet N° EP 0 437 393 fait état d'un procédé et d'un photobloréacteur destinés à la production et à l'extraction d'anti-oxydants à partir d'une culture de micro-organismes, le procédé consistant à cultiver dans le photobioréacteur des micro-algues en suspension dans un milieu de culture en collectant l'oxygène produit par les micro-algues par photosynthèse pour le réinjecter ensuite dans le milieu de culture, à séparer les micro-algues du milieu de culture, à les mettre en solution, à broyer la solution, à ajouter un solvant pour solubiliser les anti-oxydants et à séparer les phases liquides en présence.

Le brevet N° EP 0 628 629 décrit un procédé de production et d'extraction de superoxyde-dismutases thermostables à partir d'une culture de micro-organismes suspendus dans un milieu de culture et choisis parmis les micro-algues et les cyanobactéries, ce procédé consistant à cultiver dans un photoréacteur des micro-organismes thermophiles photosynthétiques aérobies producteurs d'oxygène, à extraire du milieu de culture les superoxyde-dismutases par broyage cellulaire, ultrafiltration et précipitation sélective.

Toutefois les techniques mises en oeuvre présentent l'inconvénient d'être complexes et de conduire au rejet du milieu de culture desdites micro-algues.

La présente invention est basée sur la découverte des propriétés inattendues du milieu de culture des micro-algues, qui peut dans certaines conditions produire des quantités importantes de superoxydismutases like (SOD like) et de polysaccharides sulfatés (PS).

Les superoxydismutases like (SOD like) sont des produits qui présentent le même type d'activité que les superoxydismutases (SOD), avec l'avantage sur ces dernières d'être thermostables, ce qui n'est pas le cas des enzymes en général, et des superoxydismutases en particulier.

La présente invention a ainsi pour objet un procédé d'obtention d'un extrait du milieu de culture de micro-algues, ce procédé se caractérisant essentiellement en ce qu'il consiste à réaliser dans premier temps la culture desdites micro-algues puis à leur imposer dans un deuxième temps une sursaturation en oxygène qui produit un forçage métabolique conduisant à une surproduction de composés antioxydants.

Les micro-algues mises en oeuvre dans le procédé selon l'invention sont préférentiellement choisies dans le groupe des Rhodophycae, et notamment le Porphyridium cruentum (PC).

Conformément à l'invention, la culture des micro-algues choisies s'effectue dans un photobioréacteur de type classique, dans lequel l'énergie solaire ou un éclairage approprié permet de réaliser la photosynthèse à la base de ladite culture, dans des conditions contrôlées de température de pH et d'alimentation en dioxyde de carbone CO₂.

L'alimentation en CO₂ du milieu de culture, ainsi que l'agitation nécessaire au bon développement des micro-algues, peuvent être assurées par un bullage d'air enrichi en CO₂ comprimé ou par tout autre moyen équivalent.

Au bout d'un temps de culture suffisamment long, de l'ordre de 6 à 12 jours, lorsque la densité cellulaire du milieu de culture est optimale, on y ajoute 100 mg/l de bicarbonate de potassium, puis on ferme le réacteur et on laisse la culture se poursuivre pendant un temps de l'ordre de 1 à 3 jours afin de créer un forçage métabolique provoqué par la sursaturation du milieu en oxygène provenant de la photosynthèse, le bicarbonate de potassium apportant le carbone nécessaire à cette photosynthèse.

Les algues sont ensuite séparées du milieu de culture par centrifugation ou filtration. Une filtration à l'aide d'un filtre approprié permet ensuite de séparer l'extrait anti-oxydant.

Le filtre utilisé pour séparer l'extrait recherché peut être un membrane cellulosique à dimensions de pores de 1 à 1,5 µm.

Le produit ainsi extrait du milieu de culture des micro-algues renferme au moins 30 unités par millilitre de SOD like, mesure effectuée à l'aide du kit commercialisé sous la dénomination "SOD-525". Il renferme en outre au moins 1 mg/ml de polysaccharides sulfatés.

La teneur élevée en SOD like de l'extrait selon l'invention lui confère des propriétés anti-inflammatoires qui peuvent trouver une application en rhumatologie, ainsi que des propriétés antiradicalaires qui peuvent être utilisées en cosmétique, dans la préparation de crèmes contre le vieillissement cutané.

D'autre part la teneur importante en polysaccharides sulfatés de l'extrait selon l'invention lui confère des propriétés de régénération tissulaire qui, associées aux propriétés antiradicalaires des SOD like, en font un excellent produit pro-cicatrisant.

La produit selon l'invention peut également être utilisé pour renforcer les activités anti-oxydantes d'autres extraits végétaux, notamment dans la lutte biologique contre les parasites végétaux. Ainsi des produits à activité péroxydasique peuvent bénéficier d'une activité nettement accrue par addition d'environ 10% de l'extrait selon l'invention.

Le produit selon l'invention présent l'avantage d'être thermostable, notamment à 121°C pendant vingt minutes.

Selon l'application envisagée pour l'extrait selon l'invention, on peut l'utiliser tel qu'obtenu selon le procédé décrit ci-dessus. Toutefois ont peut aussi séparer les composés actifs de manière à obtenir deux produits à activités différents, l'un contenant les SOD like et l'autre les polysaccharides sulfatés.

La séparation de la partie renfermant les SOD like peut s'effectuer par précipitation à l'aide d'un solvant tel que l'éthanol ou par séparation à l'aide d'une membrane organique telle qu'une membrane de cellulose, de dimensions de pores comprises entre 1 000 et 50 000 daltons. On obtient deux extraits dont l'un, renfermant les SOD like, présente une activité anti-oxydante et antiradicalaire, tandis que l'autre renfermant les polysaccharides sulfatés, présente une activité de régénération tissulaire.

La présente invention sera mieux comprise à la lumière de exemples qui suivent, fournis à titre de simple illustration de l'invention, vis-à-vis de laquelle ils ne présentent aucun caractère limitatif.

### EXEMPLE 1

### Obtention d'un extrait du milieu de culture de Porphyridium cruentum (PC)

Dans un photobioréacteur de 150 litres, l'algue est cultivée dans un milieu type Conway-eau de mer artificielle. L'inoculation est faite par 5 l de culture à 5 millions de cellules par millilitre. Un bullage d'air est assuré par l'énergie solaire ou par des lampes distribuant 100 µ moles/m2/s.

Au bout de 12 jours, lorsque la densité cellulaire est optimale, on rajoute 100 mg par litre, soit 15 g de KHCO3, puis on ferme le réacteur et on laisse la culture se poursuivre pendant 2 jours afin de créer un forçage métabolique.

Après ce temps, les algues sont séparées par centrifugation du milieu de culture, puis ce dernier est filtré sur membrane cellulosique à pores de 1,2 µm.

L'extrait ainsi obtenu présente une teneur en 800 like de 30 U/ml, mesurée au kit SOD-525, et une teneur en polysaccharides sulfatés de 1 mg/ml.

Cet extrait peut être stérilisé à 121°C pendant 20 minutes, puis conditionné en flacons stériles, sans que cette opération modifie l'activité SOD, ni la teneur en PS.

Il peut aussi être déshydraté par lyophilisation pour être ensuite utilisé dans la préparation de compositions tels que des comprimés.

### EXEMPLE 2

### Séparation des activités

A partir de 10 litres de l'extrait obtenu à l'exemple 1, on sépare par traitement à l'éthanol la partie SOD like des polysaccharides sulfatés, qui précipitent.

On obtient ainsi deux extraits, dont l'un présente une activité antiradicalaire non altérée par la séparation, et l'autre renferme les polysaccharides sulfatés et présente une activité de régénération tissulaire.

Chacun des deux extraits peut être conservé sous forme aqueuse, après stérilisation et conditionnement en flacons stériles.

Les extraits peuvent aussi être conservés sous forme de poudre soluble, après lyophilisation.

### EXEMPLE 3

### PRODUITS COMÉTIQUES

### 1) Crème anti-viellissement cutané.

Dans un malaxeur approprié on réalise le mélange suivant, exprimé en pourcentage en poids :

| | |
|---|---|
| EXTRAIT selon l'exemple 1 | 10 |
| PEG-8 BEESWAX | 8 |
| OCTYDODECYL MYRISTATE | 10 |
| ISOSTEARYL ISOSTERARATE | 10 |
| SODIUM HYDROXIDE | 0,4 |
| ETHOXYDIGLYCOL | 5 |
| EAU, PARFUM, CONSERVATEUR | QSP 100 |

Une objectivation in vitro de la crème ainsi obtenue permet de constater une prolifération de fibroblastes, même en présence d'agents anti-prolifératifs introduits dans le milieu, ce qui constitue l'indice d'un effet trophique et pro-reconstituant.

### 2) Brumisateur adoucissant pour peau fragilisée

On conditionne le liquide de l'exemple 1 dans un flacon propulseur à l'azote.

### EXEMPLE 4

### Composition diététique anti-strees oxydatif

A l'aide de l'extrait obtenu à l'exemple 1 on réalise des gélules de 250 mg de poudre, destinées à la nutraceutique et à la parapharmacie.

Des essais effectués sur rats, à raison d'une administration journalière de 5 mg de poudre, ont permis d'observer une augmentation des substances antiradicalaires dans le plasma sanguin.

### EXEMPLE 5

### Collyre anti-inflammatoire

On prépare un collyre selon la formulation suivante :

| | |
|---|---|
| Gluconate de chlorhexidine | 0,005 g |
| Inosine phosphate disodique déshydraté | 0,5 g |
| Extrait liquide selon l'exemple 1 qsq | 100 ml |

Le collyre ainsi obtenu peut être utilisé dans le traitement des conjonctivites.

### EXEMPLE 6

### Produit alimentaire

A un concentré de 50 degrés Brix de tomates on ajoute 0,1% en poids de la poudre de l'exemple 1, afin d'accroître sa durée de conservation.

### EXEMPLE 7

### Composition contre les parasites végétaux

L'extrait renfermant les PS de l'exemple 2 appliqué à des végétaux comme le melon ou la vigne induit une activité péroxydasique qui est le signe d'une résistance potentielle à des parasites comme les champignons.

### EXEMPLES 8

### Application aux polymères

L'extrait obtenu à l'exemple 1 peut, après déshydratation, être incorporé à des polymères pour augmenter leur résistance à l'oxydation.

## Revendications

1. Procédé d'obtention à partir du milieu de culture de micro-algues, d'un extrait thermostable possédant une activité anti-oxydante et pro-cicatrisante, consistant à réaliser dans un premier temps la culture desdites micro-algues dans un photobioréacteur soumis à un éclairage approprié et à des conditions contrôlées de température, de pH et d'alimentation en dioxyde de carbone CO₂, à leur imposer dans un deuxième temps une sursaturation en oxygène, puis à séparer les algues du milieu de culture par centrifugation, et enfin à filtrer ledit milieu de culture sur un filtre approprié pour séparer ledit extrait,
**caractérisé en ce qu'**après une durée de six à douze jours, on ajoute au milieu de culture 100 mg/l de bicarbonate de potassium, puis on ferme le réacteur pendant une durée de un à trois jours pour réaliser la sursaturation en oxygène.

2. Procédé selon la revendication 1, **caractérisé en ce que** l'éclairage de photobioréacteur est réalisé par l'énergie solaire.

3. Procédé selon la revendication 1, **caractérisé en ce que** l'alimentation en CO₂ est réalisé par bullage d'air enrichi en CO₂ comprimé.

4. Procédé selon la revendication 1, **caractérisé en ce que** la filtration du milieu de culture après la centrifugation destinée à en séparer les algues s'effectue sur un filtre constitué d'une membrane cellulosique à dimensions de pores comprises entre 1 et 1,5 µm.

5. Extrait à propriétés anti-oxydantes et pro-cicatrisantes obtenu selon le procédé qui fait l'objet des revendications 1 à 4, **caractérisé en ce qu'**il renferme au moins 30 U/ml de superoxydismutases like (SOD like) et au moins 1 mg/ml de polysaccharides sulfatés.

6. Extrait à propriétés anti-radicalaires obtenu à partir de l'extrait selon la revendication 5 par précipitation à l'aide d'un solvant ou par séparation à l'aide d'une membrane cellulosique, **caractérisé en ce qu'**il renferme au moins 30 U/ml de SOD like.

7. Extrait à propriétés de régénération tissulaire obtenu à partir de l'extrait selon la revendication 5 par précipitation à l'aide d'un solvant ou par séparation à l'aide d'une membrane cellulosique, **caractérisé en ce qu'**il renferme au moins 1 mg/ml de polysaccharides sulfatés.

8. Extrait selon la revendication 6 ou la revendication 7, **caractérisé en ce que** la séparation est réalisée à l'aide d'une membrane cellulosique à dimensions de pores comprises entre 1 000 et 50 000 daltons.

9. Extrait selon la revendication 6 ou la revendication 7, **caractérisé en ce que** la précipitation est réalisée à l'aide d'éthanol.

10. Utilisation de l'extrait selon la revendication 5 comme anti-oxydant dans la fabrication des polymères.

11. Utilisation de l'extrait selon la revendication 5 ou la revendication 6 dans la préparation de compositions diététiques anti-stress oxydatif.

12. Utilisation de l'extrait selon la revendication, 5 ou la revendication 6 pour la conservation de produits alimentaires.

13. Utilisation de l'extrait selon la revendication 5 dans la préparation de produits de produits cosmétiques destinés à ralentir le vieillissement cutané.

14. Utilisation de l'extrait selon la revendication 5 dans la préparation de compositions anti-inflammatoires, notamment en rhumatologie.

15. Utilisation de l'extrait selon la revendication 7 dans la préparation de compositions destinées à la lutte biologique contre les parasites végétaux.

## Patentansprüche

1. Verfahren zur Herstellung eines in der Wärme stabilen Extrakts aus einer Mikro-Algen-Kultur, der eine anti-oxidierende und die Narbenbildung fördernde Wirkung besitzt, indem zunächst die Kultur der oben genannten Mikro-Algen in einem Photo-Bioreaktor unter geeigneter Beleuchtung und kontrollierten Bedingungen, wie Temperatur, pH und Zufuhr von Kohlenstoffdioxid CO2, durchgeführt wird, anschließend eine Übersättigung mit Sauerstoff stattfindet, die Algen dann durch Zentrifugieren aus dem Nährboden entfernt werden und schließlich der genannte Nährboden über einen geeigneten Filter gefiltert wird, um den genannten Extrakt zu trennen,
**gekennzeichnet dadurch, dass** dem Nährboden nach sechs bis zwölf Tagen 100 mg/l Kaliumbikarbonat zugesetzt werden und der Reaktor ein bis drei Tage geschlossen wird, um die Übersättigung mit Sauerstoff zu realisieren.

2. Verfahren gemäß Patentanspruch 1, **gekennzeichnet dadurch, dass** zur Beleuchtung des Photo-Bioreaktors Sonnenenergie verwendet wird.

3. Verfahren gemäß Patentanspruch 1, **gekennzeichnet dadurch, dass** die Versorgung mit CO₂ durch sprudelnde, mit komprimiertem CO₂ angereicherte Luftblasen erfolgt.

4. Verfahren gemäß Patentanspruch 1, **gekennzeichnet dadurch, dass** zur Filterung des Nährbodens nach dem Zentrifugieren zum Abtrennen der Algen ein Filter bestehend aus einer Zellulosemembran mit einer Porengröße von 1 bis 1,5 µm verwendet wird.

5. Extrakt mit anti-oxidierender und die Narbenbildung fördernder Wirkung, hergestellt durch das in den Patentansprüchen 1 bis 4 beschriebene Verfahren, **gekennzeichnet dadurch, dass** er mindestens 30 U/ml superoxyddismutasenartiger Substanzen (SOD like) und mindestens 1 mg/ml sulfatierte Polysaccharide enthält.

6. Extrakt mit Anti-Radikal-Eigenschaften, erhalten aus dem Extrakt gemäß Patentanspruch 5 durch Ausfällen mit Hilfe eines Lösungsmittels oder durch Trennung mit Hilfe einer Zellulosemembran, **gekennzeichnet dadurch, dass** er mindestens 30 U/ml SOD like enthält.

7. Extrakt mit geweberegenerierender Wirkung, erhalten aus dem Extrakt gemäß Patentanspruch 5 durch Ausfällen mit Hilfe eines Lösungsmittels und durch Trennung mit Hilfe einer Zellulosemembran, **gekennzeichnet dadurch, dass** er mindestens 1 mg/ml sulfatierte Polysaccharide enthält.

8. Extrakt gemäß Patentanspruch 6 oder Patentanspruch 7, **gekennzeichnet dadurch, dass** die Trennung mit einer Zellulosemembran erfolgt, deren Porengröße zwischen 1000 und 50000 Dalton beträgt.

9. Extrakt gemäß Patentanspruch 6 oder Patentanspruch 7, **gekennzeichnet dadurch, dass** die Ausfällung mit Ethanol erfolgt.

10. Verwendung des Extrakts gemäß Patentanspruch 5 als Anti-Oxidationsmittel bei der Herstellung von Polymeren.

11. Verwendung des Extrakts gemäß Patentanspruch 5 oder Patentanspruch 6 bei der Herstellung von Zusammensetzungen gegen oxidativen Stress für Diätprodukte.

12. Verwendung des Extrakts gemäß Patentanspruch 5 oder Patentanspruch 6 zur Konservierung von Nahrungsmitteln.

13. Verwendung des Extrakts gemäß Patentanspruch 5 bei der Herstellung von Kosmetikprodukten, die zur Verlangsamung der Hautalterung bestimmt sind.

14. Verwendung des Extrakts gemäß Patentanspruch 5 bei der Herstellung von entzündungshemmenden Mitteln, insbesondere in der Rheumatologie.

15. Verwendung des Extrakts gemäß Patentanspruch 7 bei der Herstellung von Zusammensetzungen, die zur biologischen Bekämpfung von Pflanzenparasiten bestimmt sind.

## Claims

1. Process for obtaining a thermostable extract from a microalgae culture medium, with antioxidant and scar healing activity, in a first stage consisting in initially creating the aforesaid microalgae culture in a photobioreactor subjected to appropriate lighting and controlled conditions of temperature, pH and carbon dioxide supply, in a second stage in imposing oxygen supersaturation on them and then separating the algae from the medium culture by centrifugation, and lastly filtering the said culture medium on a suitable filter in order to separate out the said extract,
**characterized in that** after a duration of six to twelve days, 100 mg/l of potassium bicarbonate are added to the culture, followed by closing of the reactor for one to three days to create oxygen supersaturation.

2. Process according to claim 1, **characterized in that** the photobioreactor is lit by solar energy.

3. Process according to claim 1, **characterized in that** the CO₂ is supplied by bubbling through compressed CO₂ enriched air.

4. Process according to claim 1, **characterized in that** the filtration of the culture medium after centrifugation to separate out the algae takes place on a filter made of a cellulose membrane with pore dimensions between 1 and 1.5 µm.

5. Extract with antioxidant and scar-healing properties obtained according to the process which is the subject of claims 1 to 4, **characterized in that** it contains at least 30 U/ml of superoxydismutases (SOD like) and at least 1 mg/ml of sulphated polysaccharide.

6. Extract with anti-radicalizing properties obtained from the extract according to claim 5 by precipitation using a solvent or by separation using a cellulose membrane, **characterized in that** it contains at least 30 U/ml of SOD like.

7. Extract with tissue regeneration properties obtained from the extract according to claim 5 by precipitation using a solvent or by separation using a cellulose membrane, **characterized in that** it contains at least 1 mg/ml of sulphated polysaccharides.

8. Extract according to claim 6 or claim 7, **characterised in that** separation takes place using a cellulose membrane with pore dimensions between 1000 and 50000 daltons.

9. Extract according to claim 6 or claim 7, **characterized in that** precipitation is carried out using ethanol.

10. Use of the extract according to claim 5 as antioxidant in the manufacture of polymers.

11. Use of the extract according to claim 5 or claim 6 in the preparation of oxidative dietetic anti-stress compositions.

12. Use of the extract according to the claim 5 or claim 6 for the conservation of foodstuffs.

13. Use of the extract according to claim 5 in the preparation of cosmetic products intended to slow down cutaneous ageing.

14. Use of the extract according to claim 5 in the preparation of anti-inflammatory compositions, especially in rhumatology.

15. Use of the extract according to claim 7 in the preparation of compositions intended for the biological fight against plant parasites.
